# EUROPEAN PATENT SPECIFICATION

(11) **EP 4 175 534 B1**
(45) Date of publication and mention of the grant of the patent: **03.04.2024**
(21) Application number: 21798160.4
(22) Date of filing: 01.09.2021
(51) Int. Cl.: A61B 5/00, A61B 5/11

(54) **WEARABLE TAGS**
WEARABLE-ETIKETTEN
ÉTIQUETTES POUVANT ÊTRE PORTÉES

(30) Priority: 14.09.2020 US 202063078186 P; 10.08.2021 US 202117398185
(43) Date of publication of application: 10.05.2023
(73) Proprietor: Apple Inc., Cupertino, CA 95014 (US)
(72) Inventor: GRENA, Benjamin, J., Cupertino, CA 95014 (US); GERARDI, Lauren, D., Cupertino, CA 95014 (US); GOMES, Didio, V., Cupertino, CA 95014 (US); HENROT, Camille, I., Cupertino, CA 95014 (US); HOOVER, Joshua, A., Cupertino, CA 95014 (US); HUSTED, Jennifer, N., Cupertino, CA 95014 (US); RICE, Gregory, Wilson, Cupertino, CA 95014 (US); UESATO, Lia, M., Cupertino, CA 95014 (US)
(74) Representative: Simmons & Simmons
(86) International application number: PCT/US2021/048617
(87) International publication number: WO 2022/055765

(56) References cited:
- WO-A1-2016/105166
- WO-A1-2016/105166
- WO-A1-2016/110804
- WO-A1-2016/134306
- WO-A1-2016/134306
- US-A1- 2012 118 084
- US-A1- 2012 118 084
- US-A1- 2013 060 097
- US-A1- 2016 192 856
- US-A1- 2018 042 502
- US-A1- 2018 042 502
- US-A1- 2019 298 265
- NAVID AMINI ET AL: "Accelerometer-based on-body sensor localization for health and medical monitoring applications", PERVASIVE AND MOBILE COMPUTING, vol. 7, no. 6, 21 September 2011 (2011-09-21), pages 746-760, XP028121070, ISSN: 1574-1192, DOI: 10.1016/J.PMCJ.2011.09.002 [retrieved on 2011-09-21]
- NAVID AMINI ET AL: "Accelerometer-based on-body sensor localization for health and medical monitoring applications", PERVASIVE AND MOBILE COMPUTING, vol. 7, no. 6, 21 September 2011 (2011-09-21), pages 746-760, XP028121070, ISSN: 1574-1192, DOI: 10.1016/J.PMCJ.2011.09.002 [retrieved on 2011-09-21]

## Description

### Field

This relates generally to electronic devices and, more particularly, to wearable devices.

### Background

Electronic devices are sometimes used for health-related functions such as heart rate monitoring and fitness tracking. It can be challenging to incorporate the desired range of health-related functions into an electronic device. In addition to having limited functionality, conventional devices may be bulky or may otherwise be cumbersome to use.

US2018/042502A1 discusses monitoring heart activity using a wearable device, including receiving, from the wearable device, an electrocardiograph (ECG) signal and a motion signal associated with an individual upon contact with the wearable device. In response to receiving the ECG signal and the motion signal associated with the individual, a position of the wearable device in contact with the individual is determined, and a heart activity monitoring mode for operating the wearable device, based on the position of the wearable device in contact with the individual, is determined.

Navid Amini et al, 2011, "Accelerometer-based on-body sensor localization for health and medical monitoring applications*"* discusses a technique for discovering the location of sensors on the human body, using acceleration data captured during daily activities. The technique first leverages unsupervised activity discovery to detect time intervals, in which the user is walking. Then it uses a support vector machine (SVM) to analyze the patterns and estimate the position of the device on the body.

### Summary

The invention is defined in the appended claims. Generally, in the context of the disclosure, a system may include an electronic device that communicates with one or more wearable tags. The wearable tags may be placed on different parts of a user's body or clothing and may be used for one or more health-related functions such as posture monitoring, sun exposure monitoring, physical therapy, running assistance, fall detection, and other functions. The wearable tag may have different types of sensors that gather different types of sensor data depending on the health-related function that the wearable tag is being used for. Additionally, different types of output may be provided from the wearable tag and/or from the electronic device depending on the health-related function that the wearable tag is being used for. Multiple tags may be used together to obtain relative motion or position information for different parts of the user's body.

A user may configure, control, and receive data from a wearable tag using an electronic device. The electronic device is used to determine the location of the wearable tag on the user's body and may be used to determine the desired health-related function for the wearable tag based on sensor data gathered from the wearable tag. The user may selectively change or update the health-related function that the wearable tags are used for by selecting a different function on a display in the electronic device and/or by placing the wearable tag on a different location on the user's body. There is a system comprising a wearable tag and an electronic device, as set out in claim 1. Optional features are set out in the dependent claims.

### Brief Description of the Drawings

FIG. 1 is a schematic diagram of an illustrative system including one or more wearable tags and one or more electronic devices.
FIG. 2 is a side view of an illustrative wearable tag.
FIG. 3 is a perspective view of an illustrative electronic device that may communicate with one or more wearable tags.
FIG. 4 is a front view of an illustrative electronic device displaying an image when a wearable tag is detected.
FIG. 5 is a front view of an illustrative electronic device displaying an image of wearable tag locations.
FIG. 6 is a front view of an illustrative electronic device displaying an image of different wearable tag functions.
FIG. 7 is a front view of an illustrative electronic device displaying an image with tag-related data.
FIG. 8 is a diagram of an illustrative wearable tag on a user's ankle.
FIG. 9 is a diagram of an illustrative wearable tag in the palm of a user's hand.
FIG. 10 is a diagram of an illustrative set of wearable tags on a user's leg.
FIG. 11 is a diagram of an illustrative set of wearable tags coupled to different parts of a user's body.
FIG. 12 is a flow chart of illustrative steps involved in using an electronic device that communicates with one or more wearable tags.

### Detailed Description

An electronic device may be used to provide output associated with one or more wearable tags (sometimes referred to as tags, stickers, sticker tags, health-monitoring devices, etc.). The wearable tags may be coupled to a user's body or clothing and may be used for one or more health-related functions such as physical therapy, sun exposure monitoring, fitness tracking, activity tracking, medical applications, biometric applications, wellness applications, personal training, rehabilitation, fall detection, posture monitoring, stress relief, focus, full-body motion tracking, and/or other suitable health-related functions. Wearable tags may be worn one at a time, or multiple tags may be worn at the same time on different parts of the body. A wearable tag includes two or more sensors (e.g., light sensors, motion sensors, heart rate sensors, etc.), and may include haptic output devices, light sources such as a status indicator, wireless power receiving circuitry, and/or communications circuitry for communicating with an electronic device.

An electronic device may be used to configure a wearable tag for different health-related functions. When a user wishes to set up a wearable tag for the first time or change the function of a wearable tag, the user may provide input to the electronic device. The electronic device includes control circuitry that determines the location of the wearable tag on the user's body and that determines a function for the wearable tag. The electronic device may collect tag data from the wearable tag while the wearable tag is worn on the user's body and may provide appropriate output to the user based on the collected tag data. Output may be provided from the electronic device and/or may be provided from the wearable tag.

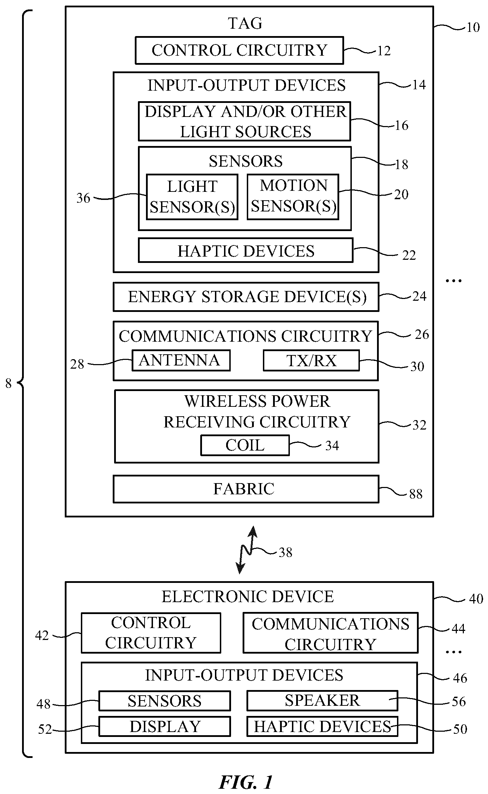

FIG. 1 is a schematic diagram of an illustrative system of the type that may include one or more wearable tags. As shown in FIG. 1, system 8 may include one or more tags such as wearable tag 10 and one or more electronic devices such as electronic device 40. Each wearable tag 10 may be worn on a person's body (e.g., a person's wrist, arm, finger, arm, neck, waist, ankle, or other suitable body part) or clothing. Tags 10 may send tag data to, receive control signals from, and/or otherwise communicate with electronic devices 40.

Tag 10 may include control circuitry 12. Control circuitry 12 may include storage and processing circuitry for supporting the operation of tag 10 and/or system 8. The storage and processing circuitry may include storage such as hard disk drive storage, nonvolatile memory (e.g., flash memory or other electrically-programmable-read-only memory configured to form a solid state drive), volatile memory (e.g., static or dynamic random-access-memory), etc. Processing circuitry in control circuitry 12 may be used to control the operation of tag 10. The processing circuitry may be based on one or more microprocessors, microcontrollers, digital signal processors, baseband processors, power management units, audio chips, application specific integrated circuits, etc.

To support interactions with external equipment, control circuitry 12 may be used in implementing communications protocols. Communications protocols that may be implemented using control circuitry 12 include internet protocols, wireless local area network protocols (e.g., IEEE 802.11 protocols -- sometimes referred to as WiFi^{®}), protocols for other short-range wireless communications links such as the Bluetooth^{®} protocol, cellular telephone protocols, MIMO protocols, antenna diversity protocols, satellite navigation system protocols, millimeter wave communications protocols, IEEE 802.15.4 ultra-wideband communications protocols, etc.

Tag 10 of system 8 may include input-output devices 14. Input-output devices 14 may be used in gathering user input, gathering health-related data, gathering information on the environment surrounding tag 10, and/or providing a user with output. Input-output devices 14 of tag 10 may include light-emitting components. For example, input-output devices 14 can include devices such as a display and/or other light sources 16. Light sources 16 may include one or more individual light-emitting devices such as light-emitting diodes, lasers, and/or lamps. Light sources 16 may include status indicator lights (e.g., a light-emitting diode that serves as a power indicator, other light-based output devices, etc.) and/or may include arrays of pixels for forming displays such as liquid crystal displays, organic light-emitting diode displays, electrophoretic displays, displays formed from Janus particles, displays formed from crystalline semiconductor dies (microLEDs), etc.

Input-output devices 14 of tag 10 include sensors 18. Sensors 18 of tag 10 are used in gathering health-related measurements. Sensors 18 of tag 10 may be used in gathering user input and may include ambient light sensors 36 (visible light sensors, color sensitive light sensors, ultraviolet light sensors, etc.), optical proximity sensors, capacitive proximity sensors, temperature sensors, force sensors (e.g., strain gauges, capacitive force sensors, resistive force sensors, force sensors for measuring biometric information, etc.), microphones for sensing audio and/or ultrasonic signals, magnetic sensors (e.g., Hall effect sensors, giant magnetoresistance sensors, or other sensors or magnetometers that measure magnetic fields), gas pressure sensors, heart rate sensors, blood oxygen level sensors (e.g., based on emitted and detected light), electrocardiogram sensors (e.g., sensors for measuring electrical signals on a user's body), humidity sensors, moisture sensors, particulate sensors (e.g., sensors that use light measurements and/or other measurements to measure particulate concentration in the air), image sensors (cameras), gas pressure sensors, carbon dioxide sensors and/or sensors measuring other gas concentrations, motion sensors 20 for detecting position, orientation, and/or movement (e.g., accelerometers, magnetic sensors such as compass sensors, gyroscopes, barometers, and/or inertial measurement units that contain some or all of these sensors), radio-frequency sensors, depth sensors (e.g., structured infrared light sensors and/or depth sensors based on stereo imaging devices), optical sensors such as self-mixing sensors and light detection and ranging (lidar) sensors that gather time-of-flight measurements, accelerometers for gathering user tap input (e.g., single taps, double taps, triple taps, etc.), and/or other sensors.

In some arrangements, tag 10 may use sensors 18 and/or other input-output devices 14 to gather user input (e.g., buttons may be used to gather button press input, touch sensors may be used in gathering touch input, microphones may be used for gathering audio input, accelerometers may be used in monitoring when a finger taps a surface and may therefore be used to gather finger tap input, etc.). Sensors 18 may include sensing electrodes, which may be formed from conductive strands of material in fabric 88 (e.g., sensor electrode pads may be formed from portions of fabric 88), may be formed from metal traces on printed circuits, and/or may be formed from other sense electrode structures.

If desired, input-output devices 14 may include one or more speakers and one or more microphones for providing tag 10 with virtual assistant functionality for a user of tag 10. For example, microphones in tag 10 may receive voice input commands and speakers in tag 10 may supply audible responses to the voice input commands, if desired.

Haptic output devices 22 may include piezoelectric devices, electromagnetic actuators, and/or other actuators for generating haptic output. Haptic output devices 22 can produce motion that is sensed by the user (e.g., through the user's skin). Haptic output devices 22 may include actuators such as electromagnetic actuators, motors, piezoelectric actuators, electroactive polymer actuators, vibrators, linear actuators, rotational actuators, actuators that bend bendable members, shape memory materials, actuator devices that create and/or control repulsive and/or attractive forces between tags 10 and/or between tag 10 and device 40 (e.g., components for creating electrostatic repulsion and/or attraction such as electrodes, components for producing ultrasonic output such as ultrasonic transducers, components for producing magnetic interactions such as electromagnets for producing direct-current and/or alternating-current magnetic fields, permanent magnets, magnetic materials such as iron or ferrite, and/or other circuitry for producing repulsive and/or attractive forces between tags 10 and/or between tag 10 and device 40). In some situations, actuators for creating forces in tag 10 may be used in producing tactile output on the user's skin.

Tag 10 may include one or more energy storage devices 24. Energy storage devices 24 may include batteries and capacitors. Capacitors for energy storage may be based on supercapacitor structures. Devices 24 may, for example, include super capacitor(s) such as electrostatic double-layer capacitors. Electrostatic double-layer capacitors (sometimes referred to as electrostatic double-layer supercapacitors) are electrochemical capacitors in which energy is stored in a capacitor formed from relatively large electrodes that are bathed in electrolyte and separated by a small distance, allowing the capacitor to achieve high energy storage capacities.

Energy storage device 24 may be charged via a wired connection or, if desired, tag 10 may charge energy storage device 24 using wirelessly received power. Power may be received wirelessly using wireless power receiving circuitry 32. Wireless power receiving circuitry 32 in tag 10 may receive power from wireless power transmitting circuitry. The wireless power transmitting circuitry may be located in device 40, may be located in a charging case (e.g., a case that stores one or more tags 10), and/or may be located in a charging mat or other electronic equipment. The wireless power transmitting circuitry may transmit power wirelessly using inductive wireless power transfer, using capacitive wireless power transfer, and/or other wireless power transfer configurations (e.g., optical).

With one illustrative configuration, wireless power receiving circuitry 32 may include, for example, inductive charging components such as coil 34 and a corresponding rectifier circuit or other wireless power receiving circuit for converting wirelessly received power from coil 34 into direct-current power for powering tag 10 and charging energy storage device 24.

As another example, wireless power receiving circuitry 32 may be configured to convert radio-frequency energy received by antenna 28 from a radio-frequency transmitter into direct-current power for powering tag 10 and charging battery 24. The radio-frequency transmitter may be located in device 40 or other electronic device and may, if desired, be located a given distance away from tag 10 (e.g., the radio-frequency transmitter need not be directly near to tag 10 for receiving circuitry 32 to be able to convert the radio-frequency energy into direct-current power).

If desired, ambient light can be converted into direct-current power for tag 10 using photovoltaic device (solar cells). Energy can also be harvested from movements of the user of tag 10 (e.g., using a piezoelectric energy harvesting device or other energy harvesting circuitry).

Control circuitry 12 may use communications circuitry 26 to transmit data to external equipment and to receive data from external equipment. Communications circuitry 26 may include wireless communication circuitry such as one or more antennas 28 and associated radio-frequency transceiver circuitry 30. Transceiver circuitry 30 may include wireless local area network transceiver circuitry (e.g., WiFi^{®} circuitry), Bluetooth^{®} circuitry, cellular telephone transceiver circuitry, ultra-wideband communications transceiver circuitry, millimeter wave transceiver circuitry, near-field communications circuitry, satellite navigation system circuitry such as Global Positioning System (GPS) receiver circuitry (e.g., for receiving GPS signals at 1575 MHz or for handling other satellite positioning data), and/or wireless circuitry that transmits and/or receives signals using light (e.g., with light-emitting diodes, lasers, or other light sources and corresponding light detectors such as photodetectors). Antennas 28 may include monopole antennas, dipole antennas, patch antennas, inverted-F antennas, loop antennas, slot antennas, other antennas, and/or antennas that include antenna resonating elements of more than one type (e.g., hybrid slot-inverted-F antennas, etc.). Antennas 28 may be formed from metal traces on printed circuits or other substrates, may include stamped metal parts, may include metal structures that form part of an enclosure or other supporting structure for tag 10, and/or other conductive structures.

Tag 10 may use communications circuitry 26 to communicate directly with device 40, to communicate with a server, and/or to communicate with other tags 10 in system 8. If desired, multiple tags 10 may be used to form nodes in a mesh network. In this type of scenario, a given tag 10 may communicate with device 40 and/or other tags 10 by routing signals through a mesh network of intermediary tags 10.

Tag 10 may include intertwined strands of material that form fabric such as fabric 88. Fabric 88 may, if desired, be stretchable fabric (e.g., elastic fabric formed using stretchable strands of material). Items such as tag 10 may therefore sometimes be referred to as fabric-based items, stretchable-fabric items, stretchable-fabric-based electronic devices, etc. In some configurations, stretchable fabric for tag 10 may form a stretchable band (e.g., a wristband, headband, armband, waistband, other stretchable band in an item of clothing, or a stretchable band that is not used as an item of clothing). Fabric 88 of tag 10 may be soft (e.g., fabric 88 may yield to a light touch), may have a rigid feel (e.g., fabric 88 may be a stiff fabric), may be coarse, may be smooth, may have ribs or other patterned textures, and/or may be formed as part of a device that has portions formed from non-fabric structures of plastic, metal, glass, crystalline materials, ceramics, or other materials.

Strands for fabric 88 may be formed from polymer, metal, glass, graphite, ceramic, natural materials as cotton or bamboo, or other organic and/or inorganic materials and combinations of these materials. Conductive coatings such as metal coatings may be formed on non-conductive material. For example, plastic yarns and monofilaments may be coated with metal to make them conductive. Reflective coatings such as metal coatings may be applied to make yarns and monofilaments reflective. Strands may be formed from a bundle of bare metal wires or metal wire intertwined with insulating monofilaments (as examples). Strands of fabric 88 may have the same color or there may be strands of two or more different colors in fabric 88, if desired.

Strands of material may be intertwined to form fabric 88 using intertwining equipment such as weaving equipment, knitting equipment, or braiding equipment. Intertwined strands may, for example, form woven fabric, knit fabric, braided fabric, etc. Conductive strands and insulating strands may be woven, knit, braided, or otherwise intertwined to form contact pads that can be electrically coupled to conductive structures in tag 10 such as the contact pads of an electrical component. The contacts of an electrical component may also be directly coupled to an exposed metal segment along the length of a conductive yarn or monofilament.

Conductive and insulating strands may also be woven, knit, or otherwise intertwined to form conductive paths. The conductive paths may be used in forming signal paths (e.g., signal buses, power lines, etc.), may be used in forming antennas, may be used in forming part of a capacitive touch sensor electrode, a resistive touch sensor electrode, a force sensor electrode, or other input-output device, or may be used in forming other patterned conductive structures. Conductive structures in the fabric of item 10 may be used in carrying power signals, digital signals, analog signals, sensor signals, control signals, data, input signals, output signals, radio-frequency signals such as antenna signals, or other suitable electrical signals.

Tag 10 may include mechanical structures in addition to fabric 88 such as polymer binder to hold strands in fabric 88 together, support structures such as frame members, housing structures (e.g., an electronic device housing), and other mechanical structures.

Additional electronic devices in system 8 such as devices 40 may include devices such as a laptop computer, a computer monitor containing an embedded computer, a tablet computer, a desktop computer, a cellular telephone, a media player, or other handheld or portable electronic device, a smaller device such as a wristwatch device, a pendant device, a headphone or earpiece device, a head-mounted device such as glasses, goggles, a helmet, or other equipment worn on a user's head, or other wearable or miniature device, a television, a computer display that does not contain an embedded computer, a gaming device, a remote control, a navigation device, an embedded system such as a system in which equipment is mounted in a kiosk, in an automobile, airplane, or other vehicle, a removable external case for electronic equipment, a strap, a wrist band or head band, a removable cover for a device, a case or bag that has straps or that has other structures to receive and carry electronic equipment and other items, a necklace or arm band, a wallet, sleeve, pocket, or other structure into which electronic equipment or other items may be inserted, part of a chair, sofa, or other seating (e.g., cushions or other seating structures), part of an item of clothing or other wearable item (e.g., a hat, belt, wrist band, headband, sock, glove, shirt, pants, etc.), or equipment that implements the functionality of two or more of these devices.

Electronic device 40 of system 8 may include control circuitry 42. Control circuitry 42 may include storage and processing circuitry for supporting the operation of device 40 and/or system 8. The storage and processing circuitry may include storage such as nonvolatile memory (e.g., flash memory or other electrically-programmable-read-only memory configured to form a solid state drive), volatile memory (e.g., static or dynamic random-access-memory), etc. Processing circuitry in control circuitry 42 may be used to gather input from sensors and other input devices and may be used to control output devices. The processing circuitry may be based on one or more microprocessors, microcontrollers, digital signal processors, baseband processors and other wireless communications circuits, power management units, audio chips, application specific integrated circuits, etc.

Electronic device 40 may include input-output devices 46. Input-output devices 46 may be used in gathering user input, in gathering information on the environment surrounding device 40, and/or in providing a user with output. Devices 46 may include sensors 48. Sensors 48 may include force sensors (e.g., strain gauges, capacitive force sensors, resistive force sensors, etc.), audio sensors such as microphones, touch and/or proximity sensors such as capacitive sensors, optical sensors such as optical sensors that emit and detect light, ultrasonic sensors, and/or other touch sensors and/or proximity sensors, monochromatic and color ambient light sensors, image sensors, sensors for detecting position, orientation, and/or motion (e.g., accelerometers, magnetic sensors such as compass sensors, gyroscopes, and/or inertial measurement units that contain some or all of these sensors), muscle activity sensors (EMG), radio-frequency sensors, depth sensors (e.g., structured light sensors and/or depth sensors based on stereo imaging devices), optical sensors such as self-mixing sensors and light detection and ranging (lidar) sensors that gather time-of-flight measurements, humidity sensors, moisture sensors, and/or other sensors. In some arrangements, device 40 may use sensors 48 and/or other input-output devices 46 to gather user input (e.g., buttons may be used to gather button press input, touch sensors overlapping displays can be used for gathering user touch screen input, touch pads may be used in gathering touch input, microphones may be used for gathering audio input, accelerometers may be used in monitoring when a finger contacts an input surface and may therefore be used to gather finger press input, etc.).

Device 40 may include haptic output devices 50. Haptic output devices 50 may include actuators such as electromagnetic actuators, motors, piezoelectric actuators, electroactive polymer actuators, vibrators, linear actuators, rotational actuators, actuators that bend bendable members, actuator devices that create and/or control repulsive and/or attractive forces between devices 10 and/or 40 (e.g., components for creating electrostatic repulsion and/or attraction such as electrodes, components for producing ultrasonic output such as ultrasonic transducers, components for producing magnetic interactions such as electromagnets for producing direct-current and/or alternating-current magnetic fields, permanent magnets, magnetic materials such as iron or ferrite, and/or other circuitry for producing repulsive and/or attractive forces between devices 10 and/or 40). In some situations, actuators for creating forces in device 40 may be used in producing tactile output (e.g., vibrations on the user's skin or that can be heard from a distance). In other situations, these components may be used to interact with each other (e.g., by creating a dynamically adjustable electromagnetic repulsion and/or attraction force between a pair of devices 40 and/or between tags 10 and device 40 using electromagnets).

If desired, input-output devices 46 of device 40 may include other devices such as display 52 (e.g., to display images for a user), status indicator lights (e.g., a light-emitting diode that serves as a power indicator, and other light-based output devices), speakers 56 and other audio output devices, electromagnets, permanent magnets, structures formed from magnetic material (e.g., iron bars or other ferromagnetic members that are attracted to magnets such as electromagnets and/or permanent magnets), batteries, etc. Device 40 may also include power transmitting and/or receiving circuits configured to transmit and/or receive wired and/or wireless power signals.

To support communications between tags 10 and device 40 and/or to support communications between equipment in system 8 and external electronic equipment, control circuitry 42 may communicate using communications circuitry 44. Circuitry 44 may include antennas, radio-frequency transceiver circuitry, and other wireless communications circuitry and/or wired communications circuitry. Circuitry 44, which may sometimes be referred to as control circuitry and/or control and communications circuitry, may, for example, support bidirectional wireless communications between devices 10 and 40 using wireless signals 38 (e.g., wireless local area network signals, near-field communications signals, Bluetooth^{®} signals, 60 GHz signals or other millimeter wave signals, ultra-wideband communications signals, etc.). Device 40 may also include power circuits for transmitting and/or receiving wired and/or wireless power and may include batteries. In configurations in which wireless power transfer is supported between tags 10 and device 40, in-band wireless communications may be supported using inductive power transfer coils (as an example).

Wireless signals 38 may be used to convey information such as location and orientation information. For example, control circuitry 42 in device 40 may determine the location of tag 10 using wireless signals 38 and/or control circuitry 12 in tag 10 may determine the location of device 40 using wireless signals 38. In one illustrative arrangement, tag 10 may include a low-power transmitter (e.g., a Bluetooth^{®} Low Energy transmitter, an ultra-wideband radio frequency signal transmitter, an RFID transmitter, a near-field communications transmitter, and/or other transmitter). Device 40 may have a corresponding receiver that detects the transmitted signals 38 from tag 10 and determines the location of tag 10 based on the received signals.

Device 40 may track the location (e.g., the indoor or outdoor location) of tag 10 using signal strength measurement schemes (e.g., measuring the signal strength of radio signals from tag 10) and/or using time-based measurement schemes such as time of flight measurement techniques, time difference of arrival measurement techniques, angle of arrival measurement techniques, triangulation methods, time-of-flight methods, using a crowdsourced location database, and other suitable measurement techniques. This type of location tracking may be achieved using ultra-wideband signals, Bluetooth^{®} signals, WiFi^{®} signals, millimeter wave signals, or other suitable signals. This is merely illustrative, however. If desired, control circuitry 42 of tag 10 may determine the distance to tag 10 using Global Positioning System receiver circuitry, using proximity sensors (e.g., infrared proximity sensors or other proximity sensors), depth sensors (e.g., structured light depth sensors that emit beams of light in a grid, a random dot array, or other pattern, and that have image sensors that generate depth maps based on the resulting spots of light produced on target objects), sensors that gather three-dimensional depth information using a pair of stereoscopic image sensors, lidar (light detection and ranging) sensors, radar sensors, using image data from a camera, using motion sensor data, and/or using other circuitry in device 40.

If desired, angle of arrival measurement techniques may be employed by control circuitry 12 of tag 10 and/or control circuitry 42 of device 40 to determine the relative orientation of tag 10 and device 40. For example, control circuitry 42 may determine the orientation of device 40 relative to tag 10 by determining a phase difference associated with signals 38 received by antennas in device 40. The phase difference may be used to determine an angle of arrival of signals 38 received by device 40. Similarly, control circuitry 12 of tag 10 may, if desired, determine the orientation of tag 10 relative to device 40 by determining a phase difference associated with signals 38 received by antennas 28 in tag 10. The phase difference may be used to determine an angle of arrival of signals 38 received by tag 10.

To keep tag 10 relatively small, lightweight, and low-power, tag 10 may include a low-power signal transmitter (e.g., a Bluetooth^{®} Low Energy transmitter, an ultra-wideband radio frequency signal transmitter, an RFID transmitter, a near-field communications transmitter, and/or other transmitter), but may not include a display or other electronics that might require a significant amount of space or power. This is merely illustrative, however. If desired, tag 10 may include a display. Arrangements in which tag 10 does not include a display are sometimes described herein as illustrative examples.

The one or more electronic devices 40 that communicate with tags 10 may sometimes be referred to as host devices. The host devices may run software that is used to track the location of tags 10, send control signals to tags 10, receive data from tags 10, and/or perform other functions related to the operation of tags 10.

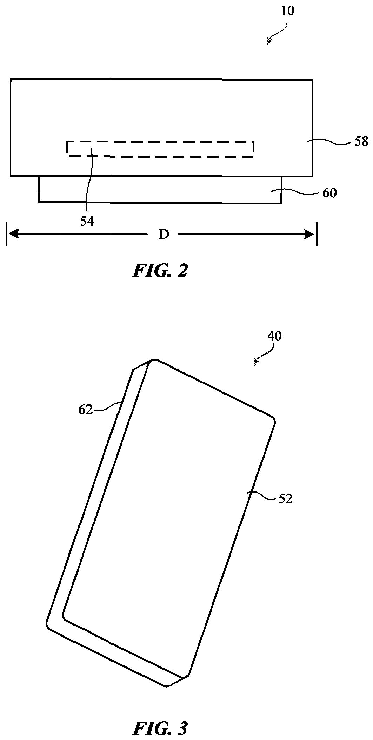

FIG. 2 is a side view of an illustrative wearable tag 10. As shown in FIG. 2, tag 10 includes a housing such as housing 58. Housing 58, which may sometimes be referred to as an enclosure or case, may be formed of plastic, glass, ceramics, fiber composites, metal (e.g., stainless steel, aluminum, etc.), fabric (e.g., fabric 88 of FIG. 1), other suitable materials, or a combination of any two or more of these materials. Housing 58 may be formed using a unibody configuration in which some or all of housing 58 is machined or molded as a single structure or may be formed using multiple structures (e.g., an internal frame structure, one or more structures that form exterior housing surfaces, etc.).

Tag 10 may include electrical components 54 mounted in housing 58. Electrical components 54 may include circuitry of the type described in connection with FIG. 1 (e.g., input-output devices 14, energy storage devices 24, communications circuitry 26, wireless power receiving circuitry 32, and/or other electrical components). Electrical components 54 may include integrated circuits, discrete components, and/or other circuits and may, if desired, be interconnected using signal paths in one or more printed circuits. If desired, one or more portions of housing 58 may be transparent to light, radio-frequency waves, and/or sound (e.g., so that light associated with a light-emitting or light-detecting component can pass through housing 58, so that radio-frequency signals can pass through housing 58, so that sound from a speaker in tag 10 can exit housing 58, so that sound from outside of tag 10 can reach a microphone in tag 10, etc.).

Tag 10 may have a circular shape, a round shape, an oval shape, a rectangular shape, and/or other suitable shape. Tag 10 may have a lateral dimension D between 25 mm and 50 mm, between 50 mm and 100 mm, between 10 mm and 200 mm, between 5 mm and 75 mm, less than 50 mm, or greater than 50 mm. The form factor of FIG. 2 is merely illustrative. In general, tag 10 may be a soft, flexible device having any suitable form factor (e.g., may be shaped as a strap or band, may be incorporated into clothing or other fabric-based item, etc.).

If desired, one or more portions of tag 10 may be formed from fabric (e.g., fabric 88 of FIG. 1). For example, a stretchy fabric may be used to hold tag 10 against a user's body so that sensors (e.g., sensor electrodes) will be in close proximity to the user's skin and can gather measurements that might otherwise be difficult or impossible to gather (e.g., skin moisture measurements, EKG measurements, blood pressure measurements, etc.). This is, however, merely illustrative. If desired, tag 10 may be formed using fabric that is not stretchable or may be formed without fabric.

Tag 10 may include attachment structure 60 for coupling tag 10 to a user's body or clothing. Attachment structure 60 may include adhesive (e.g., a semi-permanent adhesive, a skin-safe adhesive, etc.), magnets, clips, hooks, a strap or other band, and/or other structures for attaching tag 10 to the user's body or clothing.

Devices such as tag 10 of FIG. 2 may use input-output devices 14 in components 54 to gather input and provide output. As an example, tag 10 may use motion sensor(s) 20 such as accelerometers to gather motion data and analyze a user's activity (e.g., running, walking, cycling, stair climbing, hiking etc.), may use blood pressure sensors to gather blood pressure information, may use heart rate sensors to gather heart rate information, may include blood sugar sensors for gathering blood sugar levels, may use blood oxygen sensors to measure a user's blood oxygen level, may use location tracking circuitry to track the location of one part of the user's body relative to another and/or to track the user's location (e.g., location relative to another electronic device such as electronic device 40 or geographic location such as geographic coordinates), etc. If desired, accelerometers and/or other sensors may gather information on a user's respiration rate (e.g., by extracting respiration rate information from accelerometer readings). Health data, intentional user input (e.g., button press input, user input on force sensors, touch sensors, and/or other input devices, voice commands gathered with a microphone, gesture input, tap input, squeeze input, etc.), environmental readings, and/or other information on the user and the user's surroundings may be gathered by devices 14 and processed by control circuitry 12. Control circuitry 12 may also use the output devices of input-output devices 14 to provide haptic output, audio output, visual output (e.g., status light indicator output, display output such as displayed images of text, graphics, and/or video, etc.), and/or other output may be provided.

In some configurations, tag 10 may be used in isolation (e.g., as a standalone tag with input and output capabilities). In other configurations, tag 10 may operate in conjunction with external equipment (e.g., device 40 of FIG. 1). As an example, tag 10 may gather health data, location data, and/or other information using input-output devices 14 and may provide this information to device 40 via a wired or wireless communications path (e.g., wireless link 38 of FIG. 1). Device 40 can process this data and can take suitable action (e.g., using input-output devices 46 to provide output to a user or by directing tag 10 to provide output to the user using input-output circuitry 14 of tag 10).

FIG. 3 is a perspective view of an illustrative electronic device 40 that may be used to communicate with tag 10. Device 40 may include a display such as display 52. Display 52 may be mounted in a housing such as housing 62. For example, device 40 may have opposing front and rear faces and display 52 may be mounted in housing 62 so that display 52 covers the front face of device 40 as shown in FIG. 3. Housing 62, which may sometimes be referred to as an enclosure or case, may be formed of plastic, glass, ceramics, fiber composites, metal (e.g., stainless steel, aluminum, etc.), other suitable materials, or a combination of any two or more of these materials. Housing 62 may be formed using a unibody configuration in which some or all of housing 62 is machined or molded as a single structure or may be formed using multiple structures (e.g., an internal frame structure, one or more structures that form exterior housing surfaces, etc.). If desired, different portions of housing 62 may be formed from different materials. For example, housing sidewalls may be formed from metal and some or all of the rear wall of housing 62 may be formed from a dielectric such as plastic, glass, ceramic, sapphire, etc. Dielectric rear housing wall materials such as these may, if desired, by laminated with metal plates and/or other metal structures to enhance the strength of the rear housing wall (as an example).

Display 52 may be a touch screen display that incorporates a layer of conductive capacitive touch sensor electrodes or other touch sensor components (e.g., resistive touch sensor components, acoustic touch sensor components, force-based touch sensor components, light-based touch sensor components, etc.) or may be a display that is not touch-sensitive. Capacitive touch screen electrodes may be formed from an array of indium tin oxide pads or other transparent conductive structures.

Display 52 may include an array of pixels formed from liquid crystal display (LCD) components, an array of electrophoretic pixels, an array of plasma pixels, an array of organic light-emitting diode pixels, an array of electrowetting pixels, or pixels based on other display technologies.

Display 52 may be protected using a display cover layer such as a layer of transparent glass, clear plastic, sapphire, or other transparent dielectric. Openings may be formed in the display cover layer. For example, an opening may be formed in the display cover layer to accommodate a speaker and/or a button. Buttons may also be formed from capacitive touch sensors, light-based touch sensors, or other structures that can operate through the display cover layer without forming an opening.

Openings may be formed in housing 62 to form communications ports (e.g., an audio jack port, a digital data port, etc.). Openings in housing 62 may also be formed for audio components such as a speaker and/or a microphone. Dielectric-filled openings such as plastic-filled openings may be formed in metal portions of housing 62 such as in metal sidewall structures (e.g., to serve as antenna windows and/or to serve as gaps that separate portions of antennas from each other).

Tags 10 are used for health-related functions including sun exposure monitoring, fitness tracking, activity tracking, rehabilitation, fall detection, and posture monitoring. Tags 10 may be used for health-related functions such as physical therapy, medical applications, biometric applications, wellness applications, personal training, stress relief, focus, full-body tracking, and/or other suitable health-related functions. Generally speaking, the function of a given tag 10 may be set manually by the user (e.g., by providing user input to tag 10 and/or device 40) and/or may be determined automatically based on sensor data (e.g., based on where tag 10 is located on the user's body, based on how tag 10 is being used, based on how many tags 10 are coupled to the user's body or clothing, based on sensor data from sensors 18 in tag 10 and/or sensors 48 in device 40, based on health data stored in device 40, and/or based on other information). In the context of the claimed invention, the function of the tag 10 is determined automatically based on sensor data, more specifically based on sensor data from sensors 18 in device 40 indicating where the tag 10 is located on the user's body.

Generally speaking a tag 10 may have a single, set function that is predetermined during manufacturing (e.g., tag 10 may be configured specifically for posture monitoring during manufacturing), a set function that is determined during an initial set up process by the user (e.g., the user may configure tag 10 as a fitness tracking tag when setting tag 10 up for the first time) or by a third party (e.g., a physician for the user), and/or an adjustable function or set of functions that can be freely changed and reconfigured based on the user's needs. For example, the user may configure tag 10 as a fall detection device during regular use and may reconfigure tag 10 as a physical therapy assistant during physical therapy sessions. However, in the context of the claimed invention, the tag 10 is operable in at least a first mode for a first health monitoring function and a second mode for a second health monitoring function.

If desired, control circuitry 12 in tag 10 and/or control circuitry 42 in device 40 may control tag 10 differently based on the type of function being performed by tag 10. Different sensors 18 in tag 10 may be used depending on the function that tag 10 is being used for. For example, motion sensor data from one or more accelerometers and/or gyroscopes (e.g., motion sensors 20 of FIG. 1) in tag 10 may be collected when tag 10 is being used for posture monitoring, whereas light sensor data from an ultraviolet light sensor (e.g., light sensor 36 of FIG. 1) may be collected when tag 10 is being used for sun exposure monitoring. If desired, tag 10 may perform multiple health-related functions simultaneously and data from multiple sensors may be gathered and/or processed at the same time.

Different output may be provided from tag 10 and/or device 40 based on the function that tag 10 is being used for. For example, when tag 10 is configured as a fall detection device, an alert may be provided from device 40 and/or an emergency call may be made from device 40 in response to sensor data from tag 10 indicating that the user has fallen. When tag 10 is configured as a posture monitoring device and is being worn on the user's back, haptic output may be provided from haptic output devices 22 in tag 10 in response to sensor data from tag 10 indicating that the user's back is slumped. The haptic output may help remind a user to straighten his or her back. When tag 10 is configured as a sun exposure monitoring device, tag 10 and/or device 40 may provide audio output, visual output, and/or haptic output when a user has been exposed to an excessive amount of ultraviolet light. When tag 10 is being used for multiple health-related functions simultaneously, output may be provided from one or more different output devices in tag 10 and/or device 40 based on the different functions tag 10 is being used for.

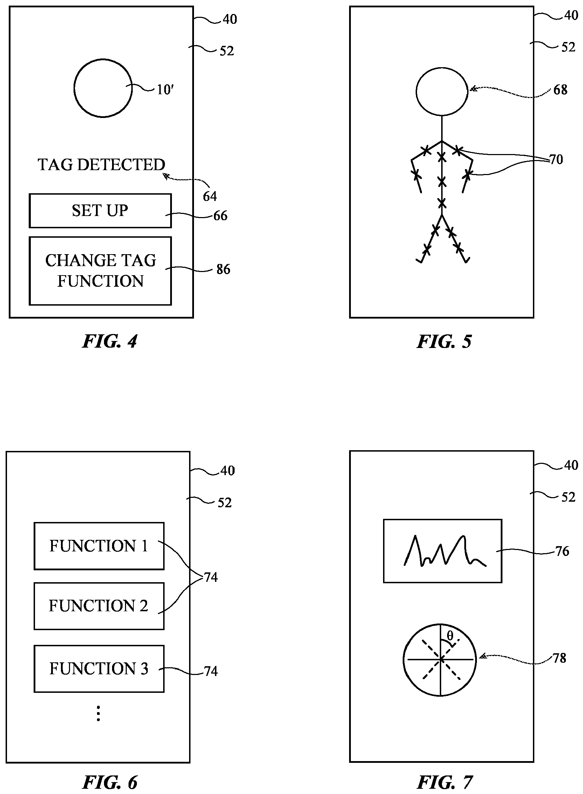

FIGS. 4, 5, 6, and 7 are illustrative screens that may be displayed on device 40 during use of tag 10.

FIG. 4 shows an illustrative screen that may be displayed when tag 10 is detected by device 40 and/or when a user provides user input to device 40 and/or tag 10 indicating the user wishes to set up or reconfigure tag 10.

Device 40 may detect tag 10 using radio-frequency signals and/or optical signals, if desired. For example, communications circuitry 44 of device 40 may receive signals 38 (FIG. 1) from a transmitter in communications circuitry 26 in tag 10. Wireless signals 38 may be used to convey information such as location and orientation information. For example, control circuitry 42 in electronic device 40 may determine the location of tags 10 using wireless signals 38 (e.g., using signal strength measurement schemes by measuring the signal strength of radio signals from tag 10, using time based measurement schemes such as time of flight measurement techniques, time difference of arrival measurement techniques, angle of arrival measurement techniques, triangulation methods, time-of-flight methods, using a crowdsourced location database, other suitable measurement techniques, etc.).

Based on signals 38 received by device 40 from tag 10, device 40 may be configured to determine the location of tag 10. The location may be a relative location (e.g., where tag 10 is located relative to device 40, where tag 10 is located on a user's body, etc.) or may be an absolute location (e.g., geographic coordinates or other absolute location). When determining the location of tag 10, device 40 may be configured to determine a distance between device 40 and tag 10 and/or may be configured to determine how device 40 is oriented with respect to tag 10 (e.g., whether device 40 is pointing towards tag 10). For example, angle of arrival measurement techniques may be used to determine the azimuth angle and/or elevation angle of tag 10 relative to a given axis of device 40 (e.g., relative to a longitudinal axis or other suitable axis of device 40) based on signals 38.

Device 40 may take action (e.g., may display an image similar to the screen of FIG. 4) when control circuitry 42 determines based on signals 38 that tag 10 is within a predetermined threshold distance from device 40 and/or when the angle of arrival of signals 38 indicates that device 40 is pointing towards tag 10 (e.g., when the azimuth angle and/or elevation angle between tag 10 and the longitudinal axis or other axis of device 40 is less than a predetermined threshold angle). Device 40 may take action automatically (e.g., without requiring user input and/or the user's attention) in response to determining that tag 10 is within a predetermined threshold distance and/or within a given threshold angle of device 40, and/or device 40 may take action after receiving manual user input (e.g., touch input, voice input, pointing input in which the user points device 40 intentionally towards tag 10, etc.).

In addition to or instead of detecting tag 10 using radio-frequency signals 38, device 40 may detect tag 10 using optical signals. Device 40 may include an image sensor (e.g., a visible light camera, an infrared camera, and/or any other suitable light sensor) that captures images of tag 10 and control circuitry 42 may be configured to detect tag 10 in the images.

In response to detecting that tag 10 is nearby (e.g., based on radio-frequency signals and/or optical signals) and/or in response to receiving user input indicating that a user wishes to set up or reconfigure a given tag 10, device 40 may present appropriate set up and/or reconfiguration options to the user. As shown in FIG. 4, for example, display 52 may display image 10' of tag 10 (e.g., an actual image of tag 10 captured by a camera, a computer-generated rendering of tag 10, and/or other suitable representation of tag 10) and/or may display text 64 indicating that tag 10 has been detected. Display 52 may display appropriate set up and or reconfiguration options such as set up option 66 and/or reconfiguration option 86 to change the tag function associated with tag 10.

When a user selects one of options 66 and/or 86, device 40 may guide the user through a set up process to help determine where tag 10 is located on the user's body and/or what tag function the user wants to assign to tag 10. For example, as shown in FIG. 5, display 52 may display a body 68 with tags in different locations 70. In one illustrative arrangement, the user may select locations 70 on body 68 (e.g., by providing touch input to display 52) to indicate where the user has placed or will place one or more tags 10. For example, the user may touch locations 70 on the left and right wrists of body 68 if the user has placed tags 10 on his or her left and right wrists. In another suitable arrangement, the user may place tags 10 on his or her body and then may use the camera in device 40 to capture images of the user and detect where tags 10 are located on the user's body. If desired, tags 10 may include a visual marker that can be detected by a visible or infrared light camera. Display 52 may be used to display an image of body 68 showing locations 70 where tags 10 have been detected by the camera. The user may optionally provide user input to confirm locations 70 and/or update locations 70 based on where tags 10 are located on the user's body.

FIG. 6 shows how display 52 may display different options 74 for illustrative tag functions that may be assigned to tag 10. Options 74 may include one, two, three, four, or more than four different options for health-related functions that can be performed by tag 10 and device 40. Options 74 may include, for example, physical therapy, sun exposure monitoring, fitness tracking, activity tracking, medical applications, biometric applications, wellness applications, personal training, rehabilitation, fall detection, posture monitoring, stress relief, focus, full-body tracking, sports applications, and/or other suitable health-related functions. The user may provide user input (e.g., touch input on display 52, voice input, etc.) to select one or more functions for tag 10. If desired, tag 10 may be set up to perform multiple functions.

FIG. 7 shows how display 52 may display tag data such as historical tag data 76 and real-time tag data 78. Historical tag data 76 may be used to show tag data (e.g., location data, motion information, posture data, fitness tracking data, heart rate information, meditation tracking, physical therapy progress, etc.) gathered by sensors 18 in tag 10 over a given period of time (e.g., during a workout or physical therapy session, over the course of a day, week, month, year, etc.). Real-time tag data 78 may be used to show current tag data in real-time. For example, a representation of the user's current posture, current heart rate, current supination or pronation angle of the foot, current flexion of the knee, and/or other real-time tag data may be presented to the user (e.g., automatically in response to certain conditions being met and/or in response to user input).

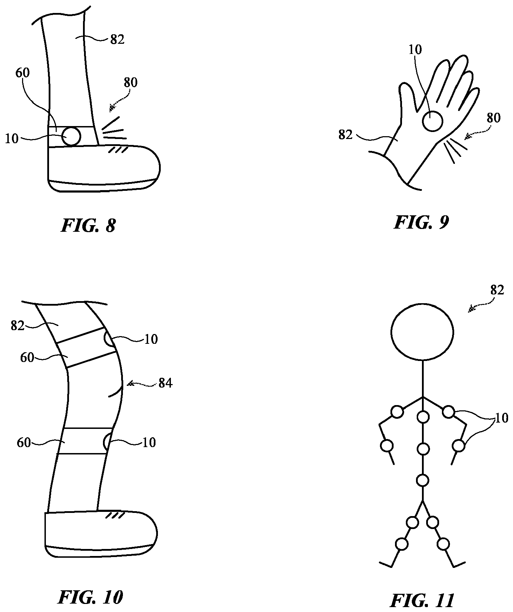

FIGS. 8, 9, 10, and 11 are illustrative examples of different use cases for tag 10. These examples are merely illustrative. If desired, tag 10 may be placed in different locations and/or may be used for other purposes.

In the example of FIG. 8, tag 10 has been placed around the ankle of user 82. Attachment structure 60 may include a strap or band that wraps around the ankle to secure tag 10 to the ankle of user 82. Tag 10 may, for example, be configured as a running assistant and may measure the supination and/or pronation angle of the user's foot (e.g., the angle between the tibia and foot) using motion sensor 20 while the user is running. Tag 10 (and/or device 40) may provide output 80 (e.g., audio output, haptic output, visual output, etc.) in response to determining based on the gathered sensor data from motion sensor 20 that the supination and/or pronation angle has exceeded a predetermined threshold angle, for example. This is merely illustrative, however. If desired, tag 10 may be used on a user's ankle or other body part for other health-related applications.

In the example of FIG. 9, tag 10 has been placed in the hand of user 82. The user may simply hold tag 10 in the palm of his or her hand or may provide input to tag 10 and/or device 10 to trigger an associated tag function. For example, the user may squeeze tag 10 and sensors 18 in tag 10 such as a force sensor (e.g., a strain gauge, piezoelectric device, or other force sensor), motion sensor 20, and/or other suitable sensor may be configured to detect the squeeze input and take suitable action. For example, the squeeze input may cause tag 10 (and/or device 40) to begin a meditation, breathing, or focus routine using output 80 (e.g., audio output, haptic output, visual output, etc.). This is merely illustrative, however. If desired, tag 10 may be used in the palm of the user's hand for other health and wellness related applications.

In the example of FIG. 10, user 82 has placed tags 10 above and below the user's knee 84. Attachment structure 60 may be a strap or other suitable structure for attaching tag 10 to the user's leg. With one tag 10 above the user's knee 84 and another tag 10 below the user's knee 84, tags 10 may be configured to measure an amount of flexion in knee 84 (e.g., by comparing the motion and/or angle of the upper leg to that of the lower leg). Tag 10 and/or device 40 may provide output (e.g., audio output, haptic output, visual output, etc.) based on the amount of flexion in knee 84 and/or based on other sensor data gathered with tag 10. This is merely illustrative, however. If desired, tag 10 may be used on a user's leg for other health-related applications.

FIG. 11 shows an illustrative example in which user 82 has placed multiple tags all over the user's body for full-body tracking capabilities. Full-body motion tracking may be used for physical therapy applications, dancing applications, fitness tracking applications, sports applications, and/or any other suitable application where full-body tracking is desired. Tag 10 and/or device 40 may provide output (e.g., audio output, haptic output, visual output, etc.) based on the motion data and other sensor data gathered by tags 10.

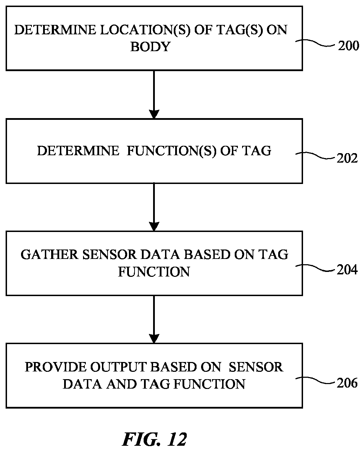

FIG. 12 is a flow chart of illustrative steps involved in operating an electronic device 40 that communicates with one or more tags 10.

During the operations of block 200, control circuitry 42 in device 40 may determine the one or more locations on the user's body where tags 10 are located. This may include, in unclaimed examples, receiving user input on locations 70 on display 52 (FIG. 5) indicating where one or more tags 10 are located on the user's body, or receiving other user input. The control circuitry 42 receives sensor data indicative of a location of the wearable tag on a body of user 82.

During the operations of block 202, control circuitry 42 may determine the function of each tag 10 on the user's body. This may include, for example, receiving user input (e.g., touch input, voice input, and/or other suitable user input) indicating what function the user wants tag 10 to perform. For example, a user may select one or more of options 74 (FIG. 6) to indicate the desired function for tag 10. If desired, the function of tag 10 may be inferred based on sensor data from tag 10. For example, the location of tag 10 on the user's body may suggest a particular function (e.g., control circuitry 42 may infer that tag 10 is intended to be used for posture monitoring if tag 10 is placed along the user's spine). Illustrative tag functions may include physical therapy, sun exposure monitoring, fitness tracking, activity tracking, medical applications, biometric applications, wellness applications, personal training, rehabilitation, fall detection, posture monitoring, stress relief, focus, full-body tracking, sports applications, and/or other suitable health-related functions.

During the operations of block 204, control circuitry 42 in device 40 may gather sensor data from tag 10. If desired, the sensor data gathered may be based on the function of tag 10 determined during the operations of block 202. For example, if the tag function is posture monitoring, control circuitry 42 may gather motion sensor data from motion sensor 20 (e.g., one or more accelerometers, gyroscopes, inertial measurement units, etc.) in tag 10 to monitor the user's posture. If tag 10 is used for sun exposure monitoring, control circuitry 42 may gather ultraviolet light measurements from light sensor 36. If tag 10 is used for fitness tracking, control circuitry 42 may gather heart rate information from a heart rate sensor in tag 10 and motion data from motion sensor 20 in tag 10. These are illustrative examples of the types of sensor data that may be gathered from tag 10 based on the one or more functions that tag 10 is being used for. In general, any suitable sensor data may be gathered depending on what function tag 10 is being used for.

During the operations of block 206, device 40 and/or tag 10 may provide output based on the sensor data gathered in step 204 and the associated tag function. For example, if tag 10 is being used for fall detection and sensor data from tag 10 indicates that the user has fallen, then device 40 and/or tag 10 may output an alert and/or make a telephone call for medical help. If tag 10 is being used for stress relief and tag data indicates that the user is stressed, device 40 and/or tag 10 may start a guided meditation program or a breathing exercise. If tag 10 is being used to monitor posture and tag data indicates that the user's posture should be corrected, device 40 and/or tag 10 may provide an audible, visual, or haptic reminder to the user to correct his or her posture. If tag 10 is being used as a running assistant to monitor foot supination or pronation and tag data indicates that the supination or pronation angle of the foot exceeds a predetermined threshold, then device 40 and/or tag 10 may provide an audible, visual, or haptic alert to the user about the foot angle. If tag 10 is being used as a rehabilitation or physical therapy tool and tag data indicates that a given goal or target has been reached, then device 40 and/or tag 10 may provide an audible, visual, or haptic notification that the user has reached the goal or target. If tag 10 is being used as one of many tags for full-body tracking for sports, dancing, or other purposes, then device 40 and/or tag 10 may provide audio, visual, and/or haptic output based on the position and movement of the body.

As described above, one aspect of the present technology is the gathering and use of information such as information from input-output devices. The present disclosure contemplates that in some instances, data may be gathered that includes personal information data that uniquely identifies or can be used to contact or locate a specific person. Such personal information data can include demographic data, location-based data, telephone numbers, email addresses, twitter ID's, home addresses, data or records relating to a user's health or level of fitness (e.g., vital signs measurements, medication information, exercise information), date of birth, username, password, biometric information, or any other identifying or personal information.

The present disclosure recognizes that the use of such personal information, in the present technology, can be used to the benefit of users. For example, the personal information data can be used to deliver targeted content that is of greater interest to the user. Accordingly, use of such personal information data enables users to have control of the delivered content. Further, other uses for personal information data that benefit the user are also contemplated by the present disclosure. For instance, health and fitness data may be used to provide insights into a user's general wellness, or may be used as positive feedback to individuals using technology to pursue wellness goals.

The present disclosure contemplates that the entities responsible for the collection, analysis, disclosure, transfer, storage, or other use of such personal information data will comply with well-established privacy policies and/or privacy practices. In particular, such entities should implement and consistently use privacy policies and practices that are generally recognized as meeting or exceeding industry or governmental requirements for maintaining personal information data private and secure. Such policies should be easily accessible by users, and should be updated as the collection and/or use of data changes. Personal information from users should be collected for legitimate and reasonable uses of the entity and not shared or sold outside of those legitimate uses. Further, such collection/sharing should occur after receiving the informed consent of the users. Additionally, such entities should consider taking any needed steps for safeguarding and securing access to such personal information data and ensuring that others with access to the personal information data adhere to their privacy policies and procedures. Further, such entities can subject themselves to evaluation by third parties to certify their adherence to widely accepted privacy policies and practices. In addition, policies and practices should be adapted for the particular types of personal information data being collected and/or accessed and adapted to applicable laws and standards, including jurisdiction-specific considerations. For instance, in the United States, collection of or access to certain health data may be governed by federal and/or state laws, such as the Health Insurance Portability and Accountability Act (HIPAA), whereas health data in other countries may be subject to other regulations and policies and should be handled accordingly. Hence different privacy practices should be maintained for different personal data types in each country.

Despite the foregoing, users may selectively block the use of, or access to, personal information data. That is, the present disclosure contemplates that hardware and/or software elements can be provided to prevent or block access to such personal information data. For example, the present technology can be configured to allow users to select to "opt in" or "opt out" of participation in the collection of personal information data during registration for services or anytime thereafter. In another example, users can select not to provide certain types of user data. In yet another example, users can select to limit the length of time user-specific data is maintained. In addition to providing "opt in" and "opt out" options, the present disclosure contemplates providing notifications relating to the access or use of personal information. For instance, a user may be notified upon downloading an application ("app") that their personal information data will be accessed and then reminded again just before personal information data is accessed by the app.

Moreover, it is the intent of the present disclosure that personal information data should be managed and handled in a way to minimize risks of unintentional or unauthorized access or use. Risk can be minimized by limiting the collection of data and deleting data once it is no longer needed. In addition, and when applicable, including in certain health related applications, data de-identification can be used to protect a user's privacy. De-identification may be facilitated, when appropriate, by removing specific identifiers (e.g., date of birth, etc.), controlling the amount or specificity of data stored (e.g., collecting location data at a city level rather than at an address level), controlling how data is stored (e.g., aggregating data across users), and/or other methods.

Therefore, although the present disclosure broadly covers use of information that may include personal information data to implement one or more various disclosed examples useful for understanding the claimed subject matter, the present disclosure also contemplates that the various examples can also be implemented without the need for accessing personal information data. That is, the various examples of the present technology are not rendered inoperable due to the lack of all or a portion of such personal information data.

## Claims

1. A system (8), comprising:
a wearable tag (10) operable in a first mode for a first health monitoring function and a second mode for a second health monitoring function, wherein the wearable tag includes first and second sensors (18) and does not include a display, and wherein the first and second health monitoring functions are different functions selected from the group consisting of a rehabilitation function, a sun exposure monitoring function, a fitness tracking function, a fall detection function, a running assistance function, and a posture monitoring function; and
an electronic device (40) having control circuitry (42) that receives sensor data indicative of a location of the wearable tag on a body of a user, wherein the control circuitry (42) collects first sensor data from the first sensor (18) when the wearable tag (10) is operated in the first mode and collects second sensor data from the second sensor (18) when the wearable tag (10) is operated in the second mode, and wherein the control circuitry (42) switches the wearable tag (10) from the first mode to the second mode based on the location of the wearable tag (10) on the body of the user.

2. The system (8) defined in claim 1 wherein the wearable tag (10) comprises wireless power receiving circuitry (32).

3. The system (8) defined in claim 1 wherein the first sensor (18) comprises a motion sensor (20).

4. The system (8) defined in claim 3 wherein the second sensor (18) comprises a light sensor (36).

5. The system (8) defined in claim 1 wherein the electronic device (40) comprises a touch-sensitive display (52) configured to receive touch input and wherein the control circuitry (42) is configured to switch the wearable tag (10) from the first mode to the second mode in response to the touch input.

6. The system (8) defined in claim 5 wherein the touch-sensitive display (52) is configured to display historical data associated with the first and second sensor data.

7. The system (8) defined in claim 5 wherein the touch-sensitive display (52) is configured to display real-time data associated with the first and second sensor data.

8. The system (8) defined in claim 1 wherein the control circuitry (42) provides a first type of output based on the first sensor data and provides a second type of output based on the second sensor data and wherein the first type of output is different from the second type of output.

9. The system (8) defined in claim 8 wherein the electronic device (40) comprises a haptic output device (50) and a display (52), and wherein the first type of output comprises haptic output from the haptic output device (50) and the second type of output comprises visual output from the display (52).

10. The system (8) defined in claim 1 wherein the first sensor (18) comprises an ultraviolet light sensor (36) and the second sensor comprises a motion sensor (20), the wearable tag (10) further comprising a haptic output device (22), wherein the control circuitry (42) is configured to:
monitor posture with the motion sensor (20);
monitor ultraviolet light exposure with the ultraviolet light sensor (36); and
provide haptic output with the haptic output device (22) based on the posture and the ultraviolet light exposure.

11. The system (8) defined in claim 10, the wearable tag (10) further comprising an ultra-wideband radio frequency signal transmitter (30).

12. The system (8) defined in claim 10, the wearable tag (10) further comprising an attachment structure selected from the group consisting of: a strap, a clip, and a magnet.

13. The system (8) defined in claim 10, the wearable tag (10) further comprising fabric (88) formed from intertwined strands of material.

## Patentansprüche

1. System (8), umfassend:
ein tragbares Etikett (10), das in einem ersten Modus für eine erste Gesundheitsüberwachungsfunktion und einem zweiten Modus für eine zweite Gesundheitsüberwachungsfunktion betriebsfähig ist, wobei das tragbare Etikett einen ersten und einen zweiten Sensor (18) einschließt und keine Anzeige einschließt, und wobei die erste und die zweite Gesundheitsüberwachungsfunktion unterschiedliche Funktionen sind, die aus der Gruppe ausgewählt sind, bestehend aus: einer Rehabilitationsfunktion, einer Sonnenexpositionsüberwachungsfunktion, einer Fitnessverfolgungsfunktion, einer Fallerfassungsfunktion, einer Laufassistenzfunktion und einer Haltungsüberwachungsfunktion; und
eine elektronische Vorrichtung (40), die eine Steuerschaltlogik (42) aufweist, die Sensordaten empfängt, hinweisend auf eine Position des tragbaren Etiketts auf einem Körper eines Benutzers, wobei die Steuerschaltlogik (42) erste Sensordaten von dem ersten Sensor (18) sammelt, wenn das tragbare Etikett (10) in dem ersten Modus betrieben wird, und zweite Sensordaten von dem zweiten Sensor (18) sammelt, wenn das tragbare Etikett (10) in dem zweiten Modus betrieben wird, und wobei die Steuerschaltlogik (42) das tragbare Etikett (10) von dem ersten Modus in den zweiten Modus basierend auf der Position des tragbaren Etiketts (10) auf dem Körper des Benutzers umschaltet.

2. System (8) nach Anspruch 1, wobei das tragbare Etikett (10) eine Drahtlosleistungsempfangsschaltlogik (32) umfasst.

3. System (8) nach Anspruch 1, wobei der erste Sensor (18) einen Bewegungssensor (20) umfasst.

4. System (8) nach Anspruch 3, wobei der zweite Sensor (18) einen Lichtsensor (36) umfasst.

5. System (8), nach Anspruch 1, wobei die elektronische Vorrichtung (40) eine berührungsempfindliche Anzeige (52) umfasst, die konfiguriert ist, um eine Berührungseingabe zu empfangen, und wobei die Steuerschaltlogik (42) konfiguriert ist, um das tragbare Etikett (10) von dem ersten Modus in den zweiten Modus als Reaktion auf die Berührungseingabe umzuschalten.

6. System (8) nach Anspruch 5, wobei die berührungsempfindliche Anzeige (52) konfiguriert ist, um historische Daten anzuzeigen, die den ersten und den zweiten Sensordaten zugeordnet sind.

7. System (8) nach Anspruch 5, wobei die berührungsempfindliche Anzeige (52) konfiguriert ist, um Echtzeitdaten anzuzeigen, die den ersten und den zweiten Sensordaten zugeordnet sind.

8. System (8) nach Anspruch 1, wobei die Steuerschaltlogik (42) eine erste Art von Ausgabe basierend auf den ersten Sensordaten bereitstellt und eine zweite Art von Ausgabe basierend auf den zweiten Sensordaten bereitstellt, und wobei sich die erste Art von Ausgabe von der zweiten Art von Ausgabe unterscheidet.

9. System (8) nach Anspruch 8, wobei die elektronische Vorrichtung (40) eine Vorrichtung mit haptischer Ausgabe (50) und eine Anzeige (52) umfasst, und wobei die erste Art von Ausgabe eine haptische Ausgabe von der Vorrichtung mit haptischer Ausgabe (50) umfasst und die zweite Art von Ausgabe eine visuelle Ausgabe von der Anzeige (52) umfasst.

10. System (8) nach Anspruch 1, wobei der erste Sensor (18) einen Ultraviolettlichtsensor (36) umfasst und der zweite Sensor einen Bewegungssensor (20) umfasst, das tragbare Etikett (10) ferner umfassend eine Vorrichtung mit haptischer Ausgabe (22), wobei die Steuerschaltlogik (42) konfiguriert ist zum:
Überwachen einer Haltung mit dem Bewegungssensor (20);
Überwachen einer Ultraviolettlichtexposition mit dem Ultraviolettlichtsensor (36); und
Bereitstellen der haptischen Ausgabe mit der Vorrichtung mit haptischer Ausgabe (22) basierend auf der Haltung und der Ultraviolettlichtexposition.

11. System (8) nach Anspruch 10, das tragbare Etikett (10) ferner umfassend einen Ultrabreitband-Hochfrequenzsignalgeber (30).

12. System (8) nach Anspruch 10, das tragbare Etikett (10) ferner umfassend eine Befestigungsstruktur, die aus der Gruppe ausgewählt ist, bestehend aus:
einem Gurt, einem Clip und einem Magneten.

13. System (8) nach Anspruch 10, das tragbare Etikett (10) ferner umfassend Gewebe (88), das aus verwebten Materialsträngen gebildet wird.

## Revendications

1. Système (8) comprenant :
une étiquette portable (10) pouvant fonctionner dans un premier mode pour une première fonction de surveillance de santé et un second mode pour une seconde fonction de surveillance de santé, dans lequel l'étiquette portable comporte des premier et second capteurs (18) et ne comporte pas un écran, et les première et seconde fonctions de surveillance de santé sont des fonctions différentes choisies dans le groupe constitué par une fonction de rééducation, une fonction de surveillance d'exposition au soleil, une fonction de suivi de condition physique, une fonction de détection de chutes, une fonction d'aide à la course et une fonction de surveillance de la posture, et
un dispositif électronique (40) ayant un circuiterie de commande (42) qui reçoit des données de capteur indiquant un emplacement de l'étiquette portable sur un corps d'un utilisateur, dans lequel la circuiterie de commande (42) collecte des premières données de capteur provenant du premier capteur (18) lorsque l'étiquette portable (10) fonctionne dans le premier mode et collecte des secondes données de capteur provenant du second capteur (18) lorsque l'étiquette portable (10) fonctionne dans le second mode, et dans lequel la circuiterie de commande (42) commute l'étiquette portable (10) du premier mode au second mode sur la base de l'emplacement de l'étiquette portable (10) sur le corps de l'utilisateur.

2. Système (8) défini dans la revendication 1, dans lequel l'étiquette portable (10) comprend une circuiterie de réception d'énergie sans fil (32).

3. Système (8) défini dans la revendication 1, dans lequel le premier capteur (18) comprend un capteur de mouvement (20).

4. Système (8) défini dans la revendication 3, dans lequel le second capteur (18) comprend un capteur de lumière (36).

5. Système (8) défini dans la revendication 1, dans lequel le dispositif électronique (40) comprend un écran tactile (52) configuré pour recevoir une entrée tactile et dans lequel la circuiterie de commande (42) est configurée pour commuter l'étiquette portable (10) du premier mode au second mode en réponse à l'entrée tactile.

6. Système (8) défini dans la revendication 5, dans lequel l'écran tactile (52) est configuré pour afficher des données historiques associées aux premières et secondes données de capteur.

7. Système (8) défini dans la revendication 5, dans lequel l'écran tactile (52) est configuré pour afficher des données en temps réel associées aux premières et secondes données de capteur.

8. Système (8) défini dans la revendication 1, dans lequel la circuiterie de commande (42) fournit un premier type de sortie sur la base des premières données de capteur et fournit un second type de sortie sur la base des secondes données de capteur et dans lequel le premier type de sortie est différent du second type de sortie.

9. Système (8) défini dans la revendication 8, dans lequel le dispositif électronique (40) comprend un dispositif de sortie haptique (50) et un écran (52), et dans lequel le premier type de sortie comprend une sortie haptique provenant du dispositif de sortie haptique (50) et le second type de sortie comprend une sortie visuelle provenant de l'écran (52).

10. Système (8) défini dans la revendication 1, dans lequel le premier capteur (18) comprend un capteur de lumière ultraviolette (36) et le second capteur comprend un capteur de mouvement (20), l'étiquette portable (10) comprenant en outre un dispositif de sortie haptique (22), dans lequel la circuiterie de commande (42) est configurée pour :
surveiller la posture avec le capteur de mouvement (20) ;
surveiller l'exposition à lumière ultraviolette avec le capteur de lumière ultraviolette (36) ; et
fournir une sortie haptique avec le dispositif de sortie haptique (22) sur la base de la posture et de l'exposition à la lumière ultraviolette.

11. Système (8) défini dans la revendication 10, l'étiquette portable (10) comprenant en outre un émetteur de signaux radiofréquence à bande ultra-large (30).

12. Système (8) défini dans la revendication 10, l'étiquette portable (10) comprenant en outre une structure de fixation sélectionnée dans le groupe constitué d'une sangle, d'une pince et d'un aimant.

13. Système (8) défini dans la revendication 10, l'étiquette portable (10) comprenant en outre un tissu (88) formé à partir de brins entrelacés de matériau.
